# EUROPÄISCHE PATENTSCHRIFT

(11) **EP 0 694 316 B1**
(45) Veröffentlichungstag und Bekanntmachung des Hinweises auf die Patenterteilung: **13.02.2002**
(21) Anmeldenummer: 95111927.0
(22) Anmeldetag: 28.07.1995
(51) Int. Cl.: A61N 5/06

(54) **Bräunungsgerät**
Tanning apparatus
Appareil de bronzage

(30) Priorität: 28.07.1994 DE 4426828
(43) Veröffentlichungstag der Anmeldung: 31.01.1996
(73) Patentinhaber: JK-JOSEF KRATZ GmbH, 53578 Windhagen (DE)
(72) Erfinder: Kratz, Walter, D-53783 Eitorf (DE)
(74) Vertreter: Heim, Hans-Karl, Dipl.-Ing.

(56) Entgegenhaltungen:
- EP-A- 0 208 392
- DE-A- 3 708 872
- DE-U- 8 518 648
- DE-U- 9 208 030

## Beschreibung

Die Erfindung betrifft ein Bräunungsgerät zur Bestrahlung einer in einem Bräunungsraum befindlichen Person mit mindestens einer Strahlungsquelle, die in einem von einem Gehäuse umschlossenen Strahlungsraum angeordnet ist, gemäß dem Oberbegriff des Anspruchs 1.

Bräunungsgeräte dieser Art sind häufig als Bräunungsliegen ausgebildet, wobei unterhalb der Liegefläche Strahlungsquellen, vorzugsweise Strahlungsröhren, angeordnet sind, die durch die transparente Liegefläche hindurch den Körper einer zu bräunenden Person bestrahlen. Die Bräunungsgeräte können zusätzlich mit einem abschwenkbaren Oberteil versehen sein, um eine gleichzeitige Bestrahlung der Körpervorder- und -rückseite zu ermöglichen. Es gibt ebenfalls Bräunungsgeräte, die nur zur Bräunung bestimmter Körperteile, z.B. des Gesichts, bestimmt sind. Weiter gibt es sogenannte Bräunungsduschen, bei denen die Strahler senkrecht um einen Bräunungsraum angeordnet sind.

Bei Ganzkörperbräunern werden UV-Strahler verwendet, die im wesentlichen die gesamte Länge der Vorrichtung einnehmen und damit eine gleichmäßige Bräunung des gesamten Körpers ermöglichen. Um eine intensivere Bräunung im Gesicht zu ermöglichen, werden bisweilen im Oberteil zusätzliche Hochdruckbräuner angeordnet, die auf das Gesicht des Benutzers gerichtet sind.

Obwohl Hersteller der UV-Strahlungsröhren und Hochdruckbräuner bemüht sind, das abgestrahlte Spektrum möglichst auf den gewünschten Spektralbereich einzuengen und höhere Verlustleistungen zu vermeiden, die zu einer Erwärmung des Geräts führen, wird der Körper der zu bräunenden Person dennoch einer hohen Wärmebelastung ausgesetzt.

Bisher hat man versucht, durch Kühleinrichtungen, wie z.B. Ventilatoren, die Wärmebelastung des Körpers zu reduzieren. Hierbei wird der Körper der zu bräunenden Person sanft angeblasen, und die erwärmte Abluft entweicht durch die meist offene Kopf- bzw. Fußseite des Geräts. Die Abstrahlwärme wird somit durch den geöffneten Innenraum des Bräunungsgeräts an die Raumluft abgegeben. Hierdurch kann die Raumtemperatur stark ansteigen, insbesondere wenn in gewerblichen Einrichtungen, wie z.B. Bräunungsstudios, mehrere Bräunungsgeräte in einem Raum angeordnet sind. Im Sommer kann die Raumtemperatur dabei bis zu Temperaturen von 40°C ansteigen.

Diese Problematik wird dadurch immer bedeutsamer, daß bei leistungsfähigen Gewerbegeräten die Anschlußwerte immer weiter gesteigert wurden, so daß zur Kühlung ein immer größerer Luftbedarf erforderlich wurde. So ergibt sich z.B. für ein Gerät mit einer Leistung von 15 bis 20 KW ein Luftdurchfluß von etwa 6.000 m³/h. Für ein Bräunungsstudio mit 8 bis 10 Geräten ergeben sich somit 40.000 bis 50.000 m³/h. Derartige Luftdurchflüsse erfordern große Rohrquerschnitte, eine entsprechende Leistung der Luftumwälzeinrichtungen, wie z.B. Gebläse, die wiederum mit einer entsprechenden Geräuschentwicklung verbunden ist.

Zur Lösung dieses Problems ist es aus dem deutschen Gebrauchsmuster G 9208030.8 bekannt, einen klimatisierten Raum vorzusehen, in dem die Liegefläche und die Strahlungsquelle angeordnet sind, wobei der klimatisierte Raum im wesentlichen abgeschlossen ist, ein Wärmetauscher in dem Bräunungsgerät zur Kühlung des klimatisierten Raums vorgesehen ist und der Wärmetauscher mit einem Wärmeträger oder Kühlmittelkreislauf verbunden ist, der als Kühlmittel Wasser verwendet.

Hierbei wurde von der Überlegung ausgegangen, mittels der Kühlung sowohl das Bräunungsgerät selbst, d.h. die Strahler, als auch den Bräunungsraum zu kühlen. Es hat sich allerdings gezeigt, daß eine derartige Klimatisierung bzw. Kühlung aufgrund der zur Verfügung stehenden Wassertemperaturen mit einfachen Mitteln nicht möglich ist.

Aus der DE 85 18 648 U1 ist ein Bestrahlungsgerät mit einem Gehäuse bekannt, in welchem ein unterer Strahlungsraum und gleichzeitig wenigstens eine Kühleinrichtung mit Gebläse angeordnet sind. Die dem unteren Strahlungsraum zugeführte Kühlluft kann in einem geschlossenen Kreislauf geführt werden. Da das Gebläse und die Kühleinrichtung in das Bestrahlungsgerät integriert sind, ist ein Wärmeübergang auf den Bräunungsraum nicht auszuschließen. Es ist deshalb vorgesehen, dass wenigstens ein Teilluftstrom über eine zusätzliche Ausgangsöffnung in der Kühleinrichtung der Umgebung bzw. dem Bräunungsraum und der zu bräunenden Person zugeführt wird. Damit verbunden ist eine erhöhte Luftumwälzung und ein größerer Energieaufwand.

Es ist die **Aufgabe** der Erfindung, ein Bräunungsgerät zu schaffen, mit dem eine problemlose Klimatisierung des Bräunungsraums und eine besonders wirksame Kühlung des Strahlungsraums ermöglicht wird.

Erfindungsgemäß wird die Aufgabe bei einem Bräunungsgerät, dessen Strahlungsraum eine geschlossene Einheit bildet, strömungsmäßig und thermisch gegenüber dem Bräunungsraum isoliert ist und zur Kühlung eine thermisch isolierte Kühleinrichtung und eine Umwälzeinrichtung zur Zuführung der Kühlluft aufweist, dadurch gelöst, dass der Strahlungsraum in einen unteren Strahlungsraum, einen seitlichen Strahlungsraum und einen oberen Strahlungsraum unterteilt ist, welche strömungsmäßig miteinander in Verbindung stehen und welchen nacheinander Luft aus der Kühleinrichtung zuführbar ist. Erfindungsgemäß ist die Kühleinrichtung außererhalb des Bräunungsraums angeordnet, wobei der Bräunungsraum den Studio- oder Kabinenraum umfasst. Zur Zu- und Abführung der Kühlluft ist eine Zu- und Ableitung vorgesehen, welche jeweils mit dem unteren Strahlungsraum bzw. dem oberen Strahlungsraum verbunden ist.

Indem der Strahlungsraum mit einem unteren, seitlichen und oberen Strahlungsraum als eine geschlossene Einheit ausgebildet ist, die sowohl strömungsmäßig als auch thermisch gegenüber dem Bräunungsraum isoliert ist, und zur Kühlung des Strahlungsraums eine von dem Bräunungsraum thermisch isolierte, außerhalb des Bräunungsraums, nämlich außerhalb des Studio- oder Kabinenraums angeordnete Kühleinrichtung vorgesehen ist, wird eine überraschend einfache, vorteilhafte Lösung erreicht.

Durch eine Umwälzeinrichtung wird die Luft aus dem oberen oder unteren Strahlungsraum durch die Kühleinrichtung geleitet und von dort dem unteren oder oberen Strahlungsraum wieder zugeführt und nacheinander durch die einzelnen Strahlungsräume geleitet.

Die Kühleinrichtung ist außerhalb des Bräunungsraums angeordnet und wird nur noch zur Kühlung des Strahlungsraums des Bräunungsgeräts verwendet. Hierdurch wird eine Erwärmung des Bräunungsraums vermieden, so daß die dort vorhandene Luft bei Bedarf auf einfache Weise klimatisiert werden kann, so daß das einfache Anblasen der zu bräunenden Person mit der Raumluft des Bräunungsraums, d.h. der Luft des Bräunungsstudios, zur Kühlung absolut ausreichend ist.

Um eine mögichst geringe Wärmebelastung des Bräunungsraums zu erreichen, ist eine Isolierung des Gehäuses des Strahlungsraums vorgesehen, da dies sonst zur Wärmeübertragung ins Studio führen und erhöhte Kühlleistungen, insbesondere an warmen Sommertagen, erfordern würde.

Vorteilhafte Ausgestaltungen der Erfindung ergeben sich aus den Unteransprüchen.

Entsprechend ist die Kühleinrichtung für die Luft aus dem Strahlungsraum als Wärmetauscher ausgebildet, der mit Luft und/oder Wasser als Kühlmittel betrieben wird. Derartige Wärmetauscher sind allgemein bekannt und erfordern weder einen großen konstruktiven noch betrieblichen Aufwand.

Gemäß einer weiteren vorteilhaften Ausgestaltung der Erfindung ist der Wärmetauscher als Verdampfungskühler ausgebildet. Derartige Verdampfungskühler zeichnen sich durch eine erhöhte Kühlleistung aus, so daß diese Ausführungsform für größere Bräunungsstudios zweckmäßig ist.

Schließlich ist der Strahlungsraum gemäß einer weiteren vorteilhaften Ausgestaltung der Erfindung in einer Modulbauweise ausgeführt, d.h. der Strahlungsraum besteht aus einzelnen zusammensetzbaren Strahlungsraumeinheiten. Dies ermöglicht eine optimierte, aerodynamische Kühlluftführung, wodurch die erforderliche elektrische Leistung für die Kühlluftumwälzung auf ca. 50 % reduziert werden kann. Mit dieser Ausführungsform wird ebenfalls eine Verminderung des Geräuschpegels von ca. 80 bis 90 % erreicht. Eine derartige Moduleinheit umfaßt etwa 10 UV-Niederdruckstrahler mit einer Leistung von jeweils 100 bis 160 W und 1 bis 2 zusätzliche UV-Hochdruckbräuner für den Gesichtsbereich mit einer Leistung von jeweils 400 bis 500 W. Selbstverständlich könnte eine derartige Einheit auch mit Zusatz-UV-Niederdruckstrahlern für den Gesichts- und Körperbräunungsbereich versehen werden. Mit dem erfindungsgemäßen Bräunungsgerät wird durch den hermetischen Abschluß des Geräteinnenraums gegen den Studioraum kein Staub in das System eingetragen, wodurch während der Bräunernutzdauer (300 bis 800 Stunden) keine Reinigung notwendig ist. Bisher mußte ein luftgekühltes Gerät etwa 10 bis 20 mal während dieser Bräunernutzdauer aufwendig gereinigt werden.

Durch eine geringere Lüfterleistung ergeben sich weiter eine erhebliche Energieeinsparung und eine Verminderung des Geräuschpegels. Das Gesamtsystem ist weniger störanfällig, da dieses erheblich weniger häufig zum Reinigen geöffnet und geschlossen werden muß. Aufgrund des verringerten Serviceaufwands ergibt sich weiter eine höhere Wirtschaftlichkeit.

Ausführungsbeispiele der vorliegenden Erfindung sind in der Zeichnung dargestellt und werden im folgenden näher beschrieben. Es zeigen:
- Fig. 1: eine schematische Ansicht des Bräunungsgeräts;
- Fig. 2: eine schematische Darstellung der Verbindung des Bräunungsgeräts mit der Kühleinrichtung in Form eines Verdampfungskühlers mit Umwälzeinrichtung; und
- Fig. 3: eine schematische Darstellung der Verbindung des Bräunungsgeräts mit der Kühleinrichtung in Form eines Wärmetauschers mit Umwälzeinrichtung.

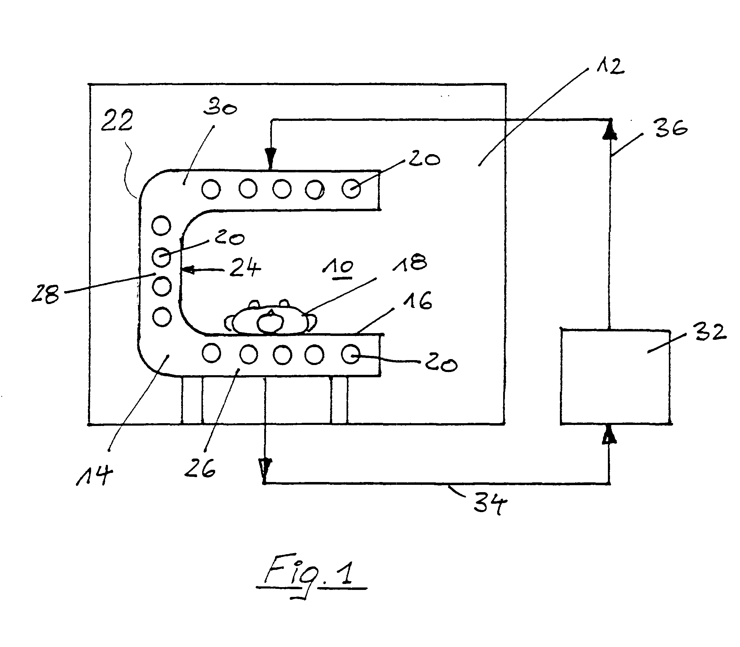

Das in Fig. 1 dargestellte Bräunungsgerät 10 umfaßt einen Bräunungsraum 12 und einen Strahlungsraum 14, wobei der Bräunungsraum 12 ebenfalls den Studio- oder Kabinenraum umfassen kann. Das dargestellte Bräunungsgerät 10 weist eine Liegefläche 16 auf, auf der die bestrahlte Person 18 liegt. Das Bräunungsgerät 10 umfaßt weiter eine Strahlungsquelle 20 in Form von mehreren UV-Niederdruckstrahlern bzw. Hochdruckstrahlern für den Gesichtsbereich, die in einem von einem Gehäuse 22 umschlossenen Strahlungsraum 24 angeordnet sind.

Der Strahlungsraum 24 kann je nach Art des Bräunungsgeräts 10 jede beliebige Form aufweisen und umfaßt im dargestellten Ausführungsbeispiel einen unteren Strahlungsraum 26 (Liege), einen seitlichen Strahlungsraum 28 und einen oberen Strahlungsraum 30 (Himmel), die strömungsmäßig miteinander in Verbindung stehen, allerdings thermisch und strömungsmäßig vom Bräunungsraum 12 isoliert sind.

Die einzelnen Strahlungsräume 26, 28, 30 können ebenfalls als Moduleinheiten ausgeführt sein, die dann entsprechend der gewünschten Größe und Form des Bräunungsgeräts 10 zusammengesetzt und strömungsmäßig für die Umwälzung der Kühlluft miteinander verbunden werden. Eine derartige Modulbauweise ermöglicht eine wesentlich bessere strömungsmäßige Führung der Kühlluft.

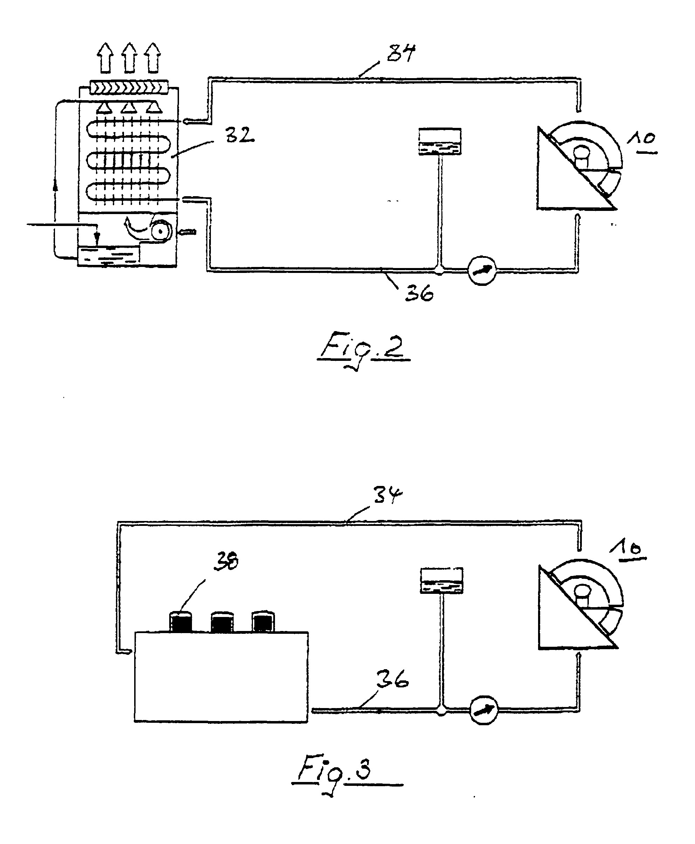

Außerhalb des Bräunungsraums 12 ist eine Kühleinrichtung 32 angeordnet, die über entsprechende Zu- und Abluftleitungen 34, 36 mit dem Strahlungsraum 24 verbunden ist. Die Kühleinrichtung 32 ist als Wärmetauscher, vorzugsweise als Verdampfungskühler, ausgebildet (siehe Fig. 2 und 3), wobei als Kühlmittel Luft, Wasser oder ein sonstiges geeignetes Kühlmittel verwendet wird. Eine geeignete Umwälzeinrichtung 38, z.B. ein Gebläse, dient zur Umwälzung der Kühlluft für den Strahlungsraum 24, d.h. das Gebläse 38 saugt die erwärmte Kühlluft aus dem Strahlungsraum 24 ab, leitet sie durch den Wärmetauscher 32 und führt die abgekühlte Luft erneut dem Strahlungsraum 24 zur Kühlung der Strahlungsquelle 20 zu.

Mit dem erfindungsgemäßen Bräunungsgerät 10 wird eine übermäßige Erwärmung des Bräunungsraums 12 vermieden, so daß die Temperatur der Umgebungsluft im Bräunungsraum 12, d.h. die Studioluft, zur angenehmen Kühlung der zu bräunenden Person 18 ausreichend ist.

## Patentansprüche

1. Bräunungsgerät zur Bestrahlung einer in einem Bräunungsraum (12) befindlichen Person,
mit mindestens einer Strahlungsquelle (20), die in einem von einem Gehäuse (22) umschlossenen Strahlungsraum (24) angeordnet ist,
der Strahlungsraum (24) bildet eine geschlossene Einheit und ist strömungsmäßig und thermisch gegenüber dem Bräunungsraum (12) isoliert,
zur Kühlung des Strahlungsraums (24) ist eine von dem Bräunungsraum (12) thermisch isolierte Kühleinrichtung (32) vorgesehen und
eine Umwälzeinrichtung (38) leitet Luft aus dem Strahlungsraum (24) durch die Kühleinrichtung (32) und von dort wieder dem Strahlungsraum (24) zu,
**dadurch gekennzeichnet,**
**dass** der Strahlungsraum (24) einen unteren Strahlungsraum (26), einen seitlichen Strahlungsraum (28) und
einen oberen Strahlungsraum (30) aufweist, welche strömungsmäßig miteinander in Verbindung stehen und welchen nacheinander Luft aus der Kühleinrichtung (32) zuführbar ist, und
**dass** die Kühleinrichtung (32) außerhalb des Bräunungsraums (12), welcher den Studio- oder Kabinenraum umfasst, angeordnet und über eine Zu- und Abflussleitung (36, 34) mit dem unteren Strahlungsraum (26) bzw. oberen Strahlungsraum (30) verbunden ist.

2. Bräunungsgerät nach Anspruch 1,
**dadurch gekennzeichnet,**
**daß** die Kühleinrichtung (32) für die Luft aus dem Strahlungsraum (24) als Wärmetauscher ausgebildet ist.

3. Bräunungsgerät nach Anspruch 1 oder 2,
**dadurch gekennzeichnet,**
**daß** als Kühlmittel für die Luft aus dem Strahlungsraum (24) Luft vorgesehen ist.

4. Bräunungsgerät nach einem der Ansprüche 1 bis 3,
**dadurch gekennzeichnet,**
**daß** als Kühlmittel für die Luft aus dem Strahlungsraum (24) Wasser vorgesehen ist.

5. Bräunungsgerät nach einem der Ansprüche 1 bis 4,
**dadurch gekennzeichnet,**
**daß** der Wärmetauscher (32) zur Kühlung der Luft aus dem Strahlungsraum (24) als Verdampfungskühler ausgebildet ist.

6. Bräunungsgerät nach einem der Ansprüche 1 bis 5,
**dadurch gekennzeichnet,**
**daß** der Strahlungsraum (24) in Modulbauweise ausgeführt ist.

## Claims

1. Tanning apparatus for irradiating a person located in a tanning area (12),
with at least one radiation source (20), which is located in a radiation area (24) surrounded by a housing (22),
the radiation area (24) forms a closed unit and is flowwise and thermally insulated from the tanning area (12),
for cooling the radiation area (24) is provided a cooling device (32) thermally insulated against the tanning area (12) and
a circulating device (38) conducts air from the radiation area (24) through the cooling device (32) and from there back to the radiation area (24),
**characterized in that**
the radiation area (24) has a lower radiation area (26), a lateral radiation area (28) and an upper radiation area (30), which are flowwise interconnected and to which can be successively supplied air from the cooling device (32) and the cooling device (32) is located outside the tanning area (12), which includes the studio or cabin area, and is connected by means of an inflow and outflow line (36, 34) to the lower radiation area (26) or upper radiation area (30).

2. Tanning apparatus according to claim 1,
**characterized in that**
the cooling device (32) for the air from the radiation area (24) is constructed as a heat exchanger.

3. Tanning apparatus according to claim 1 or 2,
**characterized in that**
air is used as coolant for the air from the radiation area (24).

4. Tanning apparatus according to one of the claims 1 to 3,
**characterized in that**
water is used as coolant for the air from the radiation area (24).

5. Tanning apparatus according to one of the claims 1 to 4,
**characterized in that**
the heat exchanger (32) for cooling the air from the radiation area (24) is constructed as an evaporative cooler.

6. Tanning apparatus according to one of the claims 1 to 5,
**characterized in that**
the radiation area (24) has a modular construction.

## Revendications

1. Appareil de bronzage pour l'exposition d'une personne se trouvant dans une chambre de bronzage (12),
avec au moins une source de rayonnement (20) qui est placée dans une chambre (24) de rayonnement entourée par une enceinte (22),
la chambre (24) de rayonnement forme une unité close et est isolée en termes de flux et thermiquement par rapport à la chambre de bronzage (12),
pour le refroidissement de la chambre (24) de rayonnement, il est prévu un dispositif de refroidissement (32) isolé thermiquement de la chambre de bronzage (12), et
un dispositif de circulation (38) extrait de l'air de la chambre (24) de rayonnement à travers le dispositif de refroidissement (32) et, de là, le renvoie à la chambre (24) de rayonnement,
***caractérisé***
***en ce que*** la chambre (24) de rayonnement présente une chambre inférieure (26) de rayonnement, une chambre latérale (28) de rayonnement et une chambre supérieure (30) de rayonnement, qui communiquent les unes avec les autres en termes de flux et auxquelles est envoyé successivement de l'air depuis le dispositif de refroidissement (32), et
***en ce que*** le dispositif de refroidissement (32) est placé à l'extérieur de la chambre de bronzage (12), laquelle comprend la salle de studio ou de cabine, et est relié par une conduite d'arrivée ou de départ (36, 34) à la chambre inférieure (26) de rayonnement ou à la chambre supérieure (30) de rayonnement.

2. Appareil de bronzage selon la Revendication 1, ***caractérisé en ce que*** le dispositif de refroidissement (32) pour l'air provenant de la chambre (24) de rayonnement est conformé en échangeur de chaleur.

3. Appareil de bronzage selon la Revendication 1 ou 2, ***caractérisé en ce que,*** comme réfrigérant pour l'air provenant de la chambre (24) de rayonnement, il est prévu de l'air.

4. Appareil de bronzage selon l'une quelconque des Revendications 1 à 3, ***caractérisé en ce que,*** comme réfrigérant pour l'air provenant de la chambre (24) de rayonnement, il est prévu de l'eau.

5. Appareil de bronzage selon l'une quelconque des Revendications 1 à 4, ***caractérisé en ce que*** l'échangeur de chaleur (32) servant à refroidir l'air provenant de la chambre (24) de rayonnement est conformé en refroidisseur à évaporation.

6. Appareil de bronzage selon l'une quelconque des Revendications 1 à 5, ***caractérisé en ce que*** la chambre (24) de rayonnement est réalisée en construction modulaire.
